# EUROPEAN PATENT APPLICATION

(11) **EP 1 496 075 A1**
(43) Date of publication of application: **12.01.2005**
(21) Application number: 04015949.3
(22) Date of filing: 07.07.2004
(51) Int. Cl.: C08G 59/40, C09D 163/02, C08L 63/02, C07C 249/02

(54) **Epoxy resin compositions**

(30) Priority: 09.07.2003 US 615694
(71) Applicant: AIR PRODUCTS AND CHEMICALS, INC., Allentown, PA 18195-1501 (US)
(72) Inventor: Chiba, Yasuo, Fujimi City 354-0018 (JP); Morimoto, Hiroya, 28-15, Honkomagome 1-chome Bunkyo Tokyo (JP); Yokomakura, Masahiro, Chiba Prefecture Nagareyama City (JP)
(74) Representative: Teipel, Susanne, Dr.

(57) **Abstract**

An epoxy resin composition is provided which has a longer working time, the so-called pot-life, and is used as adhesives, sealing agents or paints. The composition comprises a polyepoxide compound (Component A) and a ketimine (Component B) derived from the condensation reaction of an amine having 2 or more primary amino groups directed bonded to a cyclohexane ring and an aliphatic ketone.

## Description

### BACKGROUND OF THE INVENTION

This invention relates to a curable epoxy resin composition. More specifically, it is concerned with a curable epoxy resin composition having a longer pot-life, i.e. allowing a longer time for keeping the composition in workable condition for its application (or work) by maintaining an uncured state of the mixture over a given period of time, after mixing a polyepoxide and a curing agent.

The term "epoxy resin" is a generic name for chemicals having active epoxide group(s) in the molecule, and, when mixed with various active hydrogen compounds, they may be allowed to react rapidly or gradually, depending on the environment, procedures to be taken or formulations, and brought to a solidification stage. The process during which an epoxy resin composition may be structurally modified to a polymer having a three-dimensional structure is referred to as "curing". The epoxy resins have been widely applicable in semiconductor-sealing agents, printed circuit boards, paints and adhesives. In any of these fields, curability is a significant property related to quality of final products or productivity. The term "curing agent for epoxy resin" means such a compound having an active hydrogen atom, usually polyamines or acid anhydrides. A combination of an epoxy resin with a curing agent is a curable epoxy resin composition, which has been widely applied in various fields of industry such as coating agents, adhesives, sealing agents, casting agents, potting agents and sealants.

Various curing agents for epoxy resins have been used. Among them, a polyamine-type curing agent is most general. Many of amine-type curing agents are convenient in that after they are mixed with a liquid epoxy resin and simply allowed to stand in environment of room temperature or higher, a curing reaction may be initiated and proceed to a solidified stage, and thus they are widely in practical use. However, during the storage of the composition of an epoxy resin and a polyamine-type curing agent, it is necessary that an epoxide component and an amine component should be separated or in the so-called two-component system. More specifically, the epoxy resin component (main component) and the polyamine-type component (curing agent) should be stored separately and mixed immediately before use. Once the two liquids are mixed, curing reaction may be immediately initiated with increased viscosity to cause rapid or gradual solidification. In this regard, the time during which not getting to a limiting viscosity, but a certain level of viscosity is maintained so as to keep the conditions capable of performing the desired working procedures, is referred to as "pot life". A longer pot life is generally desirable because a prolonged working time can be maintained.

One of the methods adopted to prolong pot-life is a method wherein a polyamine is converted to the corresponding ketimine derivative by using a ketone compound to temporarily prevent the reactivity of the amino group in said polyamine component. More specifically, this is a method to prolong pot-life by dehydration condensation of a primary amino group in the amine molecule with a ketonic compound to form a ketimine and subsequent mixing of the ketimine with an epoxy resin as a curing agent to prevent the reactivity of the amino group.

The ketimine having the amino group of polyamine blocked with a ketone, which has been so far put to practical use in the relevant technical field for the purpose of prolonging the pot-life, is prepared using ethylenediamine, diethylenetriamine or metaxylilenediamine as a polyamine and methyl ethyl ketone or methyl isobutyl ketone as a ketone (See "New Epoxy Resins"(in Japanese), Ed. by Hiromu KAKIUTI, issued by SYOUKOUDOU, May 1985).

However, the epoxy resin system using a ketimine derived from such materials could not give practically sufficient pot-life, and there has been required an improvement in this regard.

Japanese Patent Kokai No. 217858/1996 describes a composition comprising an epoxy resin and a ketimine derivative from an aliphatic diamine, an aliphatic polyamine and an alicyclic diamine and a ketone. However, the ketimine derived by the method as described therein is an oligomer so that the derived ketimine has a high viscosity and the mixture with an epoxide has the drawback of poor workability, when used for the intended purpose.

Japanese Patent Kokai No. 60095/1998 describes a composition comprising an epoxy resin and a ketimine derived from a polyoxyalkylenepolyamine having a methyl group at the α-position thereof. In this case, a composition having a prolonged pot-life may be prepared with an epoxy resin, but it has the drawback that the starting material polyoxyalkylenepolyamine has essentially a slow curing rate and then practical applications would be restricted.

Japanese Patent Kokai No. 178343/2000 describes a composition comprising a polyepoxide and a ketimine, or a condensate of a ketone having a substituent at the α-position thereof with a polyamine having a primary amino group bonded to a secondary carbon atom. Said amine has one or two alkyl groups at the α-position thereof. All of the polyamines having such alkyl groups basically show an extremely low reactivity with an epoxide. Control of pot-life length could be accomplished, but subsequent curing may require heating or a longer time at room temperature.

Japanese Patent Kokai No. 30927/2000 describes a composition which comprises an epoxy resin and as another component a ketimine derived from a ketone having a substituent at the α-position thereof and an amine. However, the ketone having substituent at the α-position thereof as used therein is a special chemical material, so that it has the drawback in view of economical efficiency.

As explained above, when an epoxy resin is mixed with a curing agent, there has been desired development of an ideal composition which can maintain an uncured state over a prolonged period of time and can be rapidly cured when applied (or worked) as paints, adhesives or sealing agents in any manner. There is a need for an epoxy resin composition having properties of superior economical efficiency, easy handling at a low viscosity with a longer pot-life, and rapid curing after being applied (or worked).

### BRIEF SUMMARY OF THE INVENTION

An epoxy resin composition having properties of prolonged pot-life and rapid curing after application (or work) can be obtained by mixing a polyepoxide and a ketimine as a curing agent which ketimine is derived from an amine having two primary amino groups directly bonded to a cyclohexane ring. Another embodiment of the invention comprises the ketimine prepared from the condensation reaction of an amine having two primary amino groups directly bonded to a cyclohexane ring and an aliphatic ketone.

### DETAILED DESCRIPTION OF THE INVENTION

More specifically, this invention relates to a curable composition, which comprises a polyepoxide (Component A) having two or more oxirane rings per molecule, which is optionally mixed with a monoepoxide having one oxirane ring per molecule; and a ketimine as a curing agent (Component B) which is a condensation product of (a) an amine having two or more primary amino groups directly bonded to a cyclohexane ring and represented by the following general formulae 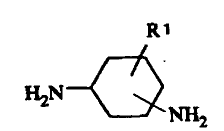 or 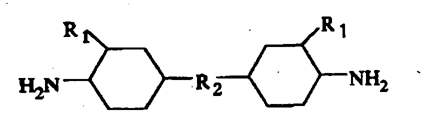 wherein R₁ is a hydrogen atom or a methyl group or an ethyl group, R₂ is a group of - CH₂-, -O- or -SO₂- or 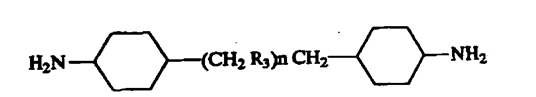 wherein R₃ is 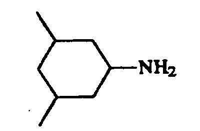 or 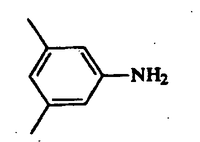 and n is an integer of 1-4 with (b) an aliphatic ketone.

The reason why the curable composition of the invention has a prolonged pot-life has not yet been completely elucidated. Ketimines used herein are formed by dehydration condensation of a primary amino group with an aliphatic ketone, and the reaction of a ketimine with an epoxy resin may be initiated by decomposition of the ketimine into the amino group and the ketone with the water in the atmosphere or from the surface of a coated product. One of the factors influencing a curing rate of the epoxy resin composition is considered to be the disturbance in approach through steric hindrance in the vicinity of the amino group against nucleophilic attack by water in the decomposition. Thus, it may be inferred that curing rate could be delayed when a ketimine converted from an amine having a bulky group is used as a curing agent for an epoxy resin. As the inference may be true of the present invention, if an amine having a bulky group such as a cyclohexyl group is converted to the corresponding ketimine for use, the ketimine is difficult to decompose owing to the steric hindrance and the period acting as amine may be prolonged, thereby giving a longer pot-life. The reason why the epoxy resin composition of the invention has a prolonged pot-life as explained above is based on mere theoretical consideration, and the technical scope of the invention is not to be limited by this theory.

The epoxy resin, Component A of the present composition, may be any of the epoxy resins reactive with an amine. The epoxy resins which may be employed in the invention are exemplified as follows:

Glycidyl ethers obtained by reacting a bisphenol-type compound such as bisphenol A, bisphenol F, bisphenol S, tetrabromobisphenol A, bisphenol hexafluoroacetone, tetramethylbisphenol A, tetramethylbisphenol F, tetrahydrobisphenol F, hexahydrobisphenol A, hydrogenated bisphenol A or hydrogenated bisphenol F with epichlorohydrin;

Glycidyl ethers obtained by reacting a novolak such as phenol novolak, cresol novolak, ethylphenol novolak, propylphenol novolak, butylphenol novolak, pentylphenol novolak, octylphenol novolak or nonylphenol novolak with epichlorohydrin;

Glycidyl ethers obtained by reacting a polyhydric phenol such as catechol, resorsine, trihydroxybiphenyl, dihydroxybenzophenone, bisresorsinol, hydroquinone, tris(hydroxyphenyl)methane, tetrakis(hydroxyphenyl)ethane or bixylenol with epichlorohydrin;

Polyglycidyl ethers obtained by reacting an aliphatic polyhydric alcohol such as glycerol, neopentyl alcohol, ethylene glycol, propylene glycol, tetramethylene glycol, hexylene glycol, polyethylene glycol or polypropylene glycol with epichlorohydrin;

Glycidyl ether esters obtained by reacting a hydroxycarboxylic acid such as p-oxybenzoic acid or β-oxynaphthoic acid with epichlorohydrin;

Polyglycidyl esters obtained by reacting a polycarboxylic acid such as phthalic acid, methylphthalic acid, isophthalic acid, terephthalic acid, tetrahydrophthalic acid, hexahydrophthalic acid, endomethylenetetrahydrophthalic acid, endomethylenehexahydrophthalic acid, trimellitic acid, a dimer acid or a polymerized aliphatic acid with epichlorohydrin;

Glycidylaminoglycidyl ethers obtained by reacting aminophenol or an aminoalkylphenol with epichlorohydrin;

Diglycidylaminoesters obtained by reacting an aminobenzoic acid with epichlorohydrin;

Polyglycidylamines obtained by reacting aniline, toluidine, 2,4,6-tribromoaniline, m-xylylenediamine, 1,2-diaminocyclohexane, 1,3-diaminocyclohexane, 1,4-diaminocyclohexane, 4,4-diaminodiphenyl ether, 4,4-diaminodiphenylmethane, 4,4-diaminodiphenylsulfone, hydantoin, an alkylhydantoin or cyanuric acid with epichlorohydrin;

Epoxidized polyolefins obtained by epoxidation of an alicyclic polyolefin or an aliphatic polyolefin; and the like.

Preferred polyepoxide compounds for use in the invention are the diglycidyl ethers of bisphenol A and bisphenol F.

The polyepoxide compounds having two or more oxirane rings per molecule may be used, if necessary, in admixture with two or more of such polyepoxide compounds.

Moreover, these polyepoxide compounds having two or more oxirane rings per molecule may be used, optionally, in admixture with a monoepoxde compound having one oxirane ring. Examples of the monoeopoxide compound include butylglycidyl ether, phenylglycidyl ether, an alkylphenylglycidyl ether, benzoic acid glycidyl ester and styrene oxide.

The ketimine, Component B of the present composition and another embodiment of the invnetion, may be obtained by a condensation reaction of (1) an amine having two or more primary amino groups directly bonded to a cyclohexane ring and having the general formulae 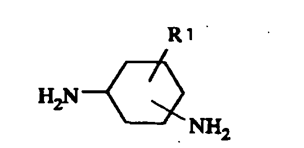 or 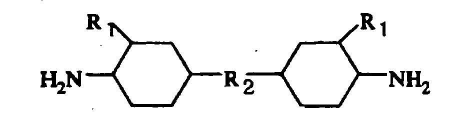 wherein R₁ is a hydrogen atom or a methyl group or an ethyl group, R₂ is a group of - CH₂-, -O- or -SO₂- or 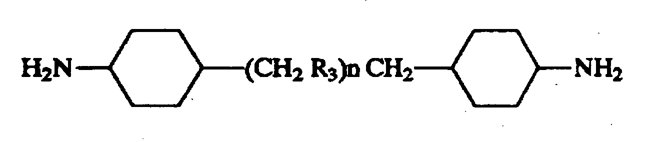 wherein R₃ is 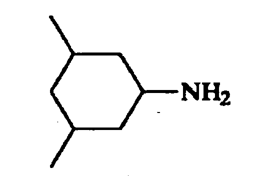 or 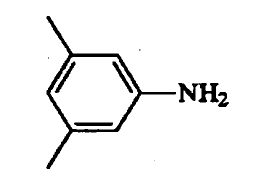 and n is an integer of 1-4 and (2) an aliphatic ketone.

Examples of the amine include 1,2-diaminocyclohexane, 1,3-diaminocyclohexane, 1,4-diaminocyclohexane, 1,2-diamino-4-methylcyclohexane, 1,3-diamino-5-methylcyclohexane, 1,4-diamino-2-methylcyclohexane, 1,2-diamino-4-ethylcyclohexane, 1,3-diamino-5-ethylcyclohexane, 1,4-diamino-2-ethylcyclohexane, bis(4-aminocyclohexyl)methane, bis(4-aminocyclohexyl)ether, bis(4-aminocyclohexyl)sulfone, bis(3-methyl-4-aminocyclohexyl)methane, bis(3-ethyl-4-aminocyclohexyl)methane, bis(3-methyl-4-aminocyclohexyl)ether, bis(3-ethyl-4-aminocyclohexyl)ether, bis(3-methyl-4-aminocyclohexyl)sulfone, bis(3-ethyl-4-aminocyclohexyl)sulfone, and an amine obtained by hydrogenation of an oligocondensate of aniline with formaldehyde. The preferred polyamine for use in making the ketimine is bis(4-aminocyclohexyl)methane also known as PACM.

Examples of the ketone which may be used as a starting material for the ketimine, Component B of the present composition, include 2-propanone (acetone), 2-butanone, 3-methyl-2-butanone, 3,3-dimethyl-2-butanone, 2-pentanone, 3-pentanone, 2-methyl-3-pentanone, 3-methyl-2-pentanone, 4-methyl-2-pentanone, 4-methyl-3-pentanone, 2,4-dimethyl-3-pentanone, 2-hexanone, 3-hexanone, 5-methyl-2-hexanone, 2-heptanone, 3-heptanone, 4-heptanone, 2-octanone and 3-octanone. The preferred ketone for use in making the ketimine is 4-methyl-2-pentanone.

A quantitative rate of Component A to Component B to be used in the present composition may be of the same level as that of the epoxy resin component to the amine component in conventional epoxy resin compositions. Component B is used in an amount corresponding to amine active hydrogen equivalent of 0.3-1.8 equivalents, preferably 0.5-1.2 equivalents, based on active amine hydrogens of the amine used to make the ketimine, relative to the epoxy group in Component A.

The ketimine, Component B of the present composition, may be obtained by reacting the above-mentioned amine with the above-mentioned ketone under general conditions. More specifically, it can be prepared by condensing amine with ketone under heating, if necessary, in the presence of an inert organic solvent and, if further necessary, a dehydrating agent, or employing azeotropic distillation, wherein one equivalent or more, for example, 1-5 equivatents, of the ketone are used for the amine, and subsequently removing an excess amount of the ketone, and optionally the solvent by azeotropic distillation and the dehydrating agent.

A curing catalyst or a curing promotor for accelerating the reaction of the epoxy compound with the ketimine may be incorporated into the composition of the invention. Examples of such agents include a monoalklphenol such as phenol, cresol, ethylphenol, propylphenol, butylphenol, octylphenol, nonylphenol; a dialkylphenol such as dimethylphenol, diethylphenol, dipropylphenol, dibutylphenol, dipentylphenol, dihexylphenol; a diphenol such as bisphenol-A or bisphenol-F; dimethylaminomethylphenol; tris(dimethylaminomethyl)phenol; salicylic acid; p-toluenesulfonic acid; phosphoric acid and derivatives thereof; and a metal salt of a carboxylic acid such as a dialkyltin laurate, a dilakyltin maleate or a dialkyltin benzoate.

The composition of the invention may comprise, together with the ketimine (the essential component B) derived from the above amines, other amines per se or as ketimine derivatives, depending on the intended purposes. Examples of such amines include propylamine, butylamine, pentylamine, hexylamine, octylamine, laurylamine, captylamine, myristylamine, stearylamine, ethlenediamine, diethylenetriamine, triethylenetetramine, tetraethylenepentamine, pentaethylenehexamine, propylenediamine, dipropylenetriamine, diaminobutane, diaminopentane, hexamethylenediamine, diaminodecane, diaminododecane, trimethylhexamethylenediamine, methylpentamethylenediamine, piperazine, aminoethylpiperazine, dimethylaminopropylamine, diethylaminopropylamine, dipropylaminopropylamine, dibutylaminopropylamine, dimethylaminoethylamine, diethylaminoethylamine, dipropylaminoethylamine, dibutylaminoethylamine, cyclohexylamine, an alkylcyclohexylamine, benzylamine, diaminocyclohexane, xylylenediamine, 1,3-bis(aminomethyl)cyclohexane, isophoronediamine, tricyclodecanediamine, benzylpropylenediamine, bis(aminomethyl)bicyclo[2.2.1]heptane, 5,5'-methylenebis-2-furanomethanediamine, a spiroacetal ring-containing diamine, menthanediamine, bis(3-methyl-4-aminocyclohexyl)methane, aniline, an alkylaniline, phenylenediamine, diethyltoluenediamine, diaminodiphenylmethane, diaminodiphenyl ether, diaminodiphenylsulfone, 1,1'-bis[4-(p-aminophenoxy)phenyl]-cyclohexane, and a benzene-formaldehyde oligomer. Moreover, there may be mentioned a mono- or polyepoxide adduct of the above amines, an amide compound with an aliphatic acid, a condensation product of phenol or an alkylphenol with formaldehyde, an adduct of acrylic acid or acrylonitrile, a ketone condensation product and the like.

A solvent for controlling viscosity may be added to the composition of the invention. Examples of the solvent include an aliphatic solvent such as heptane, hexane or cyclohexane; an aromatic solvent such as toluene, xylene, ethylbenzene or other alkylbenzenes; alcohols such as methanol, ethanol, n-propanol, isopropanol, n-butanol, isobutanol, s-butanol, ethyleneglycol, a mono- or di-alkyl ether of ethylene glycol, propylene glycol, a mono- or di-alkyl ether of propylene glycol, benzyl alcohol or cyclohexyl alcohol; or a ketone such as methyl ethyl ketone or methyl isobutyl ketone.

The following agents may be added to the composition of the invention, depending on the intended purpose. These agents include phthalate-type plasticizers such as dibutyl phthalate (DBP) or dioctyl phthalate (DOP); phosphate-type plasticizers such as triphenyl phosphate (TPP); aliphatic dibasic acid-type plasticizers such as DBA or DOS; polyester-type plasticizers from an aliphatic dibasic acid and a dihydric alcohol; and epoxidized aliphatic acid ester-type plasticizers having epoxide groups in the molecule.

Various resins may be incorporated into the composition of the invention, depending on the intended purpose. Examples of the resin include toluene-formaldehyde resins, xylene-formaldehyde resins, higher alkylbenezene-formaldehyde resins, indene-formaldehyde resins, coumarone-formaldehyde resins, and resins of copolymers thereof, phenol-formaldehyde resins, cresol-formaldehyde resins, other alkylphenol-formaldehyde resins, alkyds, polyesters, polyesteramides, polyamides, polyether esters, polyether amides, poly(vinyl alcohol), poly(vinyl acetate), poly(vinyl formal), poly(ethylene-vinyl acetate), polyacrylate, polymethacrylate, polyurethane, polyoxyethyleneglycol, polyoxypropyleneglycol, polyether polyol, vinyl polymers having a hydrolyzable silyl group or polyoxypropylene having a hydrolyzable silyl terminal group.

Moreover, various organic, inorganic or polymeric plasticizers, fillers, coloring agents, coupling agents of silane-, titanate- or aluminum-type, or solvents may be added to the composition of the invention, if necessary. Further, extender pigments and the like may be added so as to improve economic efficiency and performance of the composition.

### EXAMPLES

This invention will be more fully illustrated by way of the following examples, which do not limit the scope of the invention.

### Preparation of ketimines

The ketimines used in Examples 1-3, which were derived from amines having two or more primary amino groups directly bonded to a cyclohexane ring, and those used in Reference Examples 1-4 were prepared by the following Preparation Examples.

### Preparation Example 1

Ketimine derived from 1,2-diaminocyclohexane (DACH) and 4-methyl-2-pentanone (MIBK)

Into a 500 g-volume flask equipped with a magnetic stirrer, a thermometer, a nitrogen inlet device and Dean-Stark device were charged 57 g (0.5 mol) of 1,2-diaminocyclohexane and 250 g (2.5 mol) of 4-methyl-2-pentanone. The temperature was gradually raised up to 160°C by heating with a mantle heater, while 4-methyl-2-pentanone was refluxed. The water distilled off during that period was collected into a separate vessel. When the amount of the water collected reached about 18 g, the temperature was raised and maintained at 180°C for 2 hours to remove the remaining 4-methyl-2-pentanone. After cooling, 135 g of the desired ketimine was taken out as a residue in the flask. The product has a viscosity of 9.7 mPa·s at 25°C and a calculated active hydrogen equivalent of 67.

### Preparation Example 2

Ketimine derived from bis(aminocyclohexyl)methane (PACM) and 4-methyl-2-pentanone

Into the same equipment as in Preparation Example 1 were charged 105 g (0.5 mol) of bis(aminocyclohexyl)methane and 250 g (2.5 mol) of 4-methyl-2-pentanone and treated in the same manner as above. As a result, there was obtained 183 g of the ketimine from bis(aminocyclohexyl)-methane and 4-methyl-2-pentanone. This product has a viscosity of 180 mPa·s at 25°C and a calculated active hydrogen equivalent of 92.

### Preparation Example 3

Ketimine derived from a multifunctional polyamine (MPCA) and 4-methyl-2-pentanone

Into the same equipment as in Preparation Example 1 were charged 105 g of MPCA, which is prepared by hydrogenation of the oligomer obtained from condensation of aniline with formaldehyde and marketed under the trademark ANCAMINE 2167 curing agent from Air Products-Japan, and 250 g (2.5 mol) of 4-methyl-2-pentanone, and treated in the same manner as in Preparation Example 1. There was obtained 180 g of the ketimine of MPCA. This product has a viscosity of 200 mPa·s at 25°C, and a calculated active hydrogen equivalent of 90.

### Preparation Example 4

Ketimine derived from norbornanediamine (NBDA) and 4-methyl-2-pentanone

Into the same equipment as in Preparation Example 1 were charged 77 g (0.5 mol) of norbornanediamine and 250 g (2.5 mol) of 4-methyl-2-pentanone, and treated in the same manner as in Preparation Example 1. There was obtained 155 g of the ketimine of NBDA. This product has a viscosity of 20 mPa·s at 25°C, and a calculated active hydrogen equivalent of 80.

### Preparation Example 5

Ketimine derived from metaxylylenediamine (MXDA) and 4-methyl-2-pentanone

Into the same equipment as in Preparation Example 1 were charged 68 g (0.5 mol) of metaxylylenediamine and 250 g (2.5 mol) of 4-methyl-2-pentanone, and treated in the same manner as in Preparation Example 1. There was obtained 145 g of the ketimine of MXDA. This product has a viscosity of 8.3 mPa·s at 25°C, and a calculated active hydrogen equivalent of 75.

### Example 1

A curable epoxy resin composition was prepared by mixing 6.7 g of the ketimine prepared in Preparation Example 1 and 3 g of xylene as a solvent for controlling viscosity with 19 g of a commercially available diglycidyl ether of bisphenol A (DGEBA) (epoxy equivalent of about 190) as a polyepoxide.

### Example 2

Similarly, a curable epoxy resin composition was prepared by mixing 9.2 g of the ketimine prepared in Preparation Example 2 and 4 g of xylene as a solvent for controlling viscosity with 19 g of a commercially available diglycidyl ether of bisphenol A (DGEBA) (epoxy equivalent of about 190) as a polyepoxide.

### Example 3

Similarly, a curable epoxy resin composition was prepared by mixing 9.0 g of the ketimine prepared in Preparation Example 3 and 3 g of xylene as a solvent for controlling viscosity with 19 g of a commercially available diglycidyl ether of bisphenol A (DGEBA) (epoxy equivalent of about 190) as a polyepoxide.

Each of the epoxy resin compositions thus prepared was stored in a glass vessel under an airtight sealing and taken out of the vessel to investigate changes in viscosity with time.

Further, the same compositions were prepared separately and tested for dry-hard time by means of a dry-hard tester of RCI-type at room temperature.

### Reference Example 1

A curable epoxy resin composition was prepared by mixing 8.0 g of the ketimine prepared in Preparation Example 4 and 3 g of xylene as a solvent for controlling viscosity with 19 g of a commercially available diglycidyl ether of bisphenol A (DGEBA) (epoxy equivalent of about 190) as a polyepoxide used in Examples 1-3.

### Reference Example 2

Similarly, a curable epoxy resin composition was prepared by mixing 7.5 g of the ketimine prepared in Preparation Example 5 and 3 g of xylene as a solvent for controlling viscosity with 19 g of a commercially available diglycidyl ether of bisphenol A (DGEBA) (epoxy equivalent of about 190) as a polyepoxide.

### Reference Example 3

Similarly, a curable epoxy resin composition was prepared by mixing 5.5 g of the ketimine derived from ethylenediamine as marketed under the trademark ANCAMINE 2458 curing agent from Air Products-Japan and 2 g of xylene with 19 g of a commercially available diglycidyl ether of bisphenol A (DGEBA) (epoxy equivalent of 190) as a polyepoxide.

### Reference Example 4

Similarly, a curable epoxy resin composition was prepared by mixing 10 g of the ketimine derived from ethylenediamine as marketed under the trademark ANCAMINE 2459 curing agent from Air Products-Japan and 5 g of xylene with 19 g of a commercially available diglycidyl ether of bisphenol A (DGEBA) (epoxy equivalent of 190) as a polyepoxide.

Each of the epoxy resin compositions thus prepared was stored in a glass vessel under an airtight sealing and taken out of the vessel to investigate changes in viscosity with time in the same manner as in Examples 1-3.

Further, the same compositions were prepared separately and tested for dry-hard time by means of a dry-hard tester of RCI-type at room temperature.

The above examples will be shown in the following Table 1, in which properties of the ketimines as used in the Examples and Reference Examples, compounding formulations of the polyepoxide, the ketimine and the solvent used, days required for obtaining twice higher viscosity relative to an initial viscosity of the formulation when left to stand at room temperature, and similarly, days required for three times higher viscosity, and test results on dry-hard time at room temperature as epoxy resin compositions. Examples are represented as Examples 1-3 and reference examples are as Reference Examples 1-4.

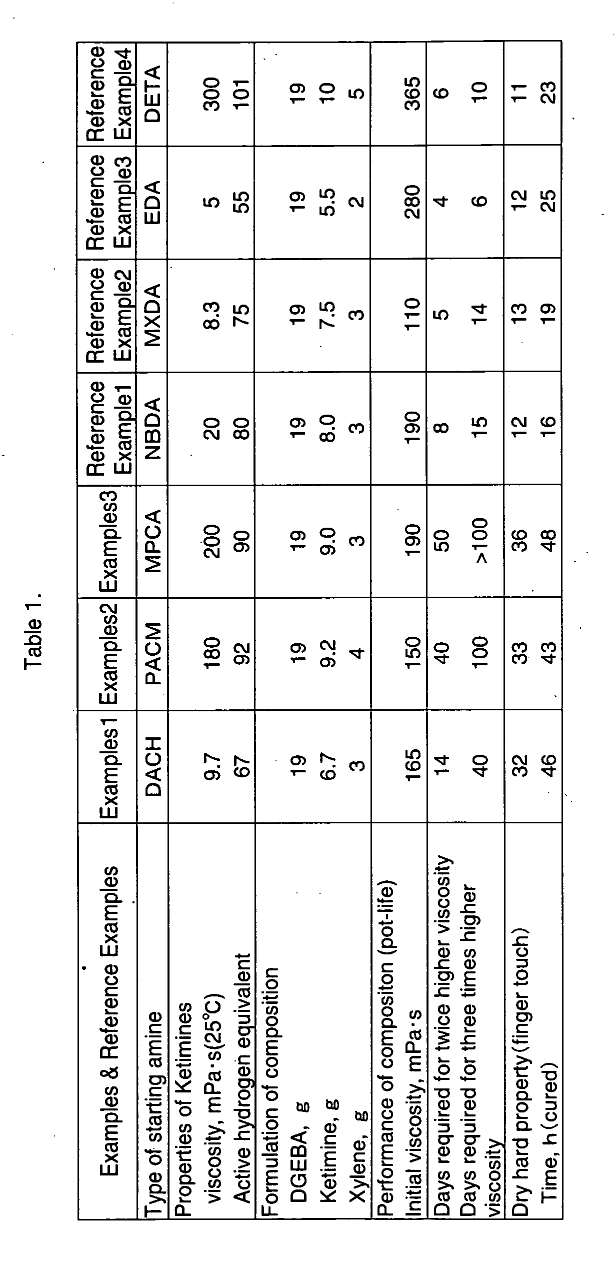

As is apparent from Table 1, the composition of the invention comprising the epoxide and the ketimine derived from an amine directly bonded to a cyclohexane ring, i.e., 1,2-diaminocyclohexane (DACH), bis(aminocyclohexyl)methane (PACM) or the polyamine (MPCA), shows a remarkably longer pot-life, that is, a longer time required for obtaining twice or three times higher viscosity, although a somewhat longer time is required in dry-hard property, as compared with the composition in which the ketimine is derived from existing products, norbornanediamine (NBDA), metaxylylenediamine (MXDA), EDA or DETA.

As explained above in detail, the present invention provides a curable epoxy resin composition, which comprises an epoxy resin as a main component and a ketimine compound derived from an amine having primary amino groups directly bonded to a cyclohexane ring as a curing agent. This curing agent enables a longer pot-life under moisture-proof environments, and after the application, an effective reaction may occur by absorbing moisture in atmosphere or from the surface of a coated material to give the desired epoxy resin cured product. The invention provides a reliable and efficient epoxy resin composition with improved workability and reliability, that is, the drawbacks of conventional epoxy resin compositions such as complicated metering and mixing, required application within a short limited time, and erroneous mixing ratio adopted *in situ* and the like are improved.

The cured products are useful as a cured resin with higher performance and functionality in various industrial fields including typically adhesives, sealing agents or paints.

## Claims

1. A curable epoxy resin composition, which comprises (a) a polyepoxide having two or more oxirane rings per molecule, which is optionally mixed with a monoepoxide having one oxirane ring per molecule; and (b) a ketimine as a curing agent which is a condensation reaction product of (1) an amine having two or more primary amino groups directly bonded to a cyclohexane ring and represented by the following general formulae 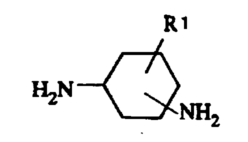 or 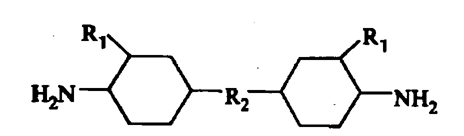 wherein R₁ is a hydrogen atom or a methyl group or an ethyl group, R₂ is a group of - CH₂-, -O- or -SO₂- or 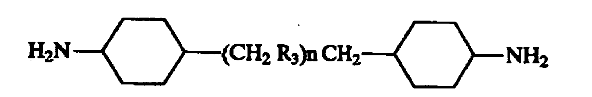 wherein R₃ is 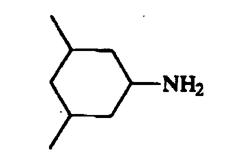 or 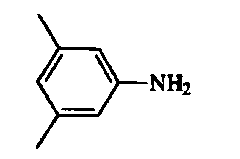 and n is an integer of 1-4 and (2) an aliphatic ketone.

2. The curable epoxy resin composition of claim 1 wherein the ketimine is a condensation reaction product of an aliphatic ketone with 1,2-diaminocyclohexane, 1,3-diaminocyclohexane, 1,4-diaminocyclohexane, 1,2-diamino-4-methylcyclohexane, 1,3-diamino-5-methylcyclohexane, 1,4-diamino-2-methylcyclohexane, 1,2-diamino-4-ethylcyclohexane, 1,3-diamino-5-ethylcyclohexane, 1,4-diamino-2-ethylcyclohexane, bis(4-aminocyclohexyl)methane, bis(4-aminocyclohexyl)ether, bis(4-aminocyclohexyl)sulfone, bis(3-methyl-4-aminocyclohexyl)methane, bis(3-ethyl-4-aminocyclohexyl)methane, bis(3-methyl-4-aminocyclohexyl)ether, bis(3-ethyl-4-aminocyclohexyl)ether, bis(3-methyl-4-aminocyclohexyl)sulfone, bis(3-ethyl-4-aminocyclohexyl)sulfone, or an amine obtained by hydrogenation of an oligocondensate of aniline with formaldehyde.

3. The curable epoxy resin composition of claim 1 wherein the ketimine is a condensation reaction product of an aliphatic ketone with an amine having two or more primary amino groups directly bonded to a cyclohexane ring, the aliphatic ketone being 2-propanone, 2-butanone, 3-methyl-2-butanone, 3,3-dimethyl-2-butanone, 2-pentanone, 3-pentanone, 2-methyl-3-pentanone, 3-methyl-2-pentanone, 4-methyl-2-pentanone, 4-methyl-3-pentanone, 2,4-dimethyl-3-pentanone, 2-hexanone, 3-hexanone, 5-methyl-2-hexanone, 2-heptanone, 3-heptanorie, 4-heptanone, 2-octanone or 3-octanone.

4. The curable epoxy resin composition of claim 1 wherein the ketimine is a condensation reaction product of an aliphatic ketone with 1,2-diaminocyclohexane, 1,3-diaminocyclohexane, 1,4-diaminocyclohexane, bis(4-aminocyclohexyl)methane, bis(3-methyl-4-aminocyclohexyl)methane or a polyamine obtained by hydrogenation of an oligocondensate of aniline with formaldehyde.

5. The curable epoxy resin composition of claim 1 wherein the ketimine is a condensation reaction product of an aliphatic ketone with an amine having two or more primary amino groups directly bonded to a cyclohexane ring, the aliphatic ketone being 2-butanone, 3,3-dimethyl-2-butanone, 2-pentanone, 3-pentanone, 2-methyl-3-pentanone, 2,4-dimethyl-3-pentanone or 5-methyl-2-hexanone.

6. The curable epoxy resin composition of claim 1 wherein the ketimine is a condensation reaction product of 4-methyl-2-pentanone with an amine having two or more primary amino groups directly bonded to a cyclohexane ring.

7. The curable epoxy resin composition claim 1 wherein the polyepoxide is a polyepoxide of glycidyl ether, glycidyl ether ester, glycidyl ester, glycidyl amine, glycidylaminoglycidyl ether, glycidylaminoglycidyl ester or epoxidized polyolefin type.

8. The curable epoxy resin composition of claim 1 wherein the polyepoxide is a glycidyl ether obtained by reacting a bisphenol-type compound with epichlorohydrin, the bisphenol-type compound being bisphenol A, bisphenol F, bisphenol S, tetrabromobisphenol A, bisphenol hexafluoroacetone, tetramethylbisphenol A, tetramethylbisphenol F, tetrahydrobisphenol F, hexahydrobisphenol A, hydrogenated bisphenol A or hydrogenated bisphenol F with epichlorohydrin;
a glycidyl ether obtained by reacting a novolak-type compound of phenol novolak, cresol novolak, ethylphenol novolak, propylphenol novolak, butylphenol novolak, pentylphenol novolak, octylphenol novolak or nonylphenol novolak with epichlorohydrin;
a glycidyl ether obtained by reacting a polyhydric phenol of catechol, resorsine, trihydroxybiphenyl, dihydroxybenzophenone, bisresorsinol, hydroquinone, tris(hydroxyphenyl)methane, tetrakis(hydroxyphenyl)ethane or bixylenol with epichlorohydrin; or
a polyglycidyl ether obtained by reacting an aliphatic polyhydric alcohol of glycerol, neopentyl alcohol, ethylene glycol, propylene glycol, tetramethylene glycol, hexylene glycol, polyethylene glycol or polypropylene glycol with epichlorohydrin:

9. The curable epoxy resin composition of claim 7 wherein the polyepoxide of glycidyl ether type is a polyglycidyl ether obtained by reacting a polyhydric hydroxy compound of bisphenol A, bisphenol F, phenol novolak, cresol novolak, octylphenol novolak or nonylphenyl novolak with epichlorohydrin; or a polyglycidyl ether obtained by reacting an aliphatic polyhydric alcohol of ethylene glycol, propylene glycol, tetramethylene glycol, neopentyl glycol, hexylene glycol, polyethylene glycol or polypropylene glycol with epichlorohydrin.

10. The curable epoxy resin composition of claim 7 wherein the polyepoxide of glycidyl ether ester type is a polyglycidyl ether ester obtained by reacting a hydroxycarboxylic acid of p-oxybenzoic acid or β-oxynaphthoic acid with epichlorohydrin.

11. The curable epoxy resin composition of claim 7 wherein the polyepoxide of glycidyl ester type is a polyglycidyl ester obtained by reacting a polycarboxylic acid of phthalic acid, methylphthalic acid, isophthalic acid, terephthalic acid, tetrahydrophthalic acid, hexahydrophthalic acid, endomethylenetetrahydrophthalic acid, endomethylenehexahydrophthalic acid, trimellitic acid, a dimer acid or a polymerized aliphatic acid with epichlorohydrin.

12. The curable epoxy resin composition of claim 7 wherein the polyepoxide of glycidylaminoglycidyl ether type is a glycidylaminoglycidyl ether obtained by reacting aminophenol or an aminoalkylphenol with epichlorohydrin.

13. The curable epoxy resin composition of claim 7 wherein the polyepoxide of glycidylaminoglycidyl ester type is a glycidylaminoglycidyl ester obtained by reacting an aminobenzoic acid with epichlorohydrin.

14. The curable epoxy resin composition of claim 7 wherein the polyepoxide of glycidylamine type is a polyglycidylamine obtained by reacting an amino compound of aniline, toluidine, 2,4,6-tribromoaniline, m-xylylenediamine, 1,2-diaminocyclohexane, 1,3-diaminocydohexane, 1,4-diaminocyclohexane, 4,4-diaminodiphenyl ether, 4,4-diaminodiphenylmethane, 4,4-diaminodiphenylsulfone, hydantoin, an alkylhydantoin or cyanuric acid with epichlorohydrin.

15. The curable epoxy resin composition of claim 7 wherein the polyepoxide of epoxidized olefin type is an epoxidized polyolefin obtained by epoxidation of an alicyclic polyolefin or an aliphatic polyolefin.

16. A ketimine which is the condensation reaction product of (1) an amine having two or more primary amino groups directly bonded to a cyclohexane ring and represented by the following general formulae 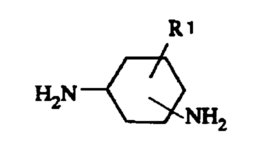 or 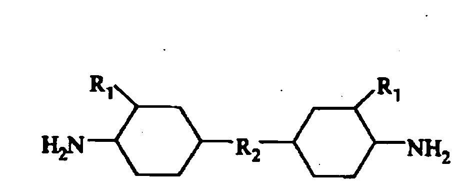 wherein R₁ is a hydrogen atom or a methyl group or an ethyl group, R₂ is a group of - CH₂-, -O- or -SO₂- or 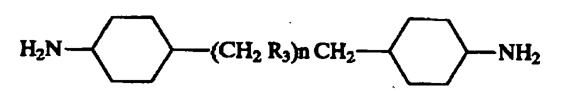 wherein R₃ is 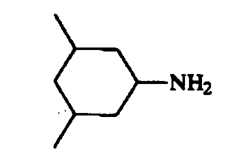 or 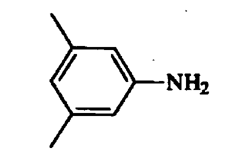 and n is an integer of 1-4 and (2) an aliphatic ketone.

17. The ketimine of Claim 16 in which the amine is 1,2-diaminocyclohexane, 1,3-diaminocyclohexane, 1,4-diaminocyclohexane, 1,2-diamino-4-methylcyclohexane, 1,3-diamino-5-methylcyclohexane, 1,4-diamino-2-methylcyclohexane, 1,2-diamino-4-ethylcyclohexane, 1,3-diamino-5-ethylcyclohexane, 1,4-diamino-2-ethylcyclohexane, bis(4-aminocyclohexyl)methane, bis(4-aminocyclohexyl)ether, bis(4-aminocyclohexyl)sulfone, bis(3-methyl-4-aminocyclohexyl)methane, bis(3-ethyl-4-aminocyclohexyl)methane, bis(3-methyl-4-aminocyclohexyl)ether, bis(3-ethyl-4-aminocyclohexyl)ether, bis(3-methyl-4-aminocyclohexyl)sulfone, bis(3-ethyl-4-aminocyclohexyl)sulfone, or an amine obtained by hydrogenation of an oligocondensate of aniline with formaldehyde and the ketone is 2-propanone, 2-butanone, 3-methyl-2-butanone, 3,3-dimethyl-2-butanone, 2-pentanone, 3-pentanone, 2-methyl-3-pentanone, 3-methyl-2-pentanone, 4-methyl-2-pentanone, 4-methyl-3-pentanone, 2,4-dimethyl-3-pentanone, 2-hexanone, 3-hexanone, 5-methyl-2-hexanone, 2-heptanone, 3-heptanone, 4-heptanone, 2-octanone or 3-octanone.

18. The ketimine of Claim 16 in which the amine is 1,2-diaminocyclohexane, 1,3-diaminocyclohexane, 1,4-diaminocyclohexane, bis(4-aminocyclohexyl)methane, bis(3-methyl-4-aminocyclohexyl)methane or a polyamine obtained by hydrogenation of an oligocondensate of aniline with formaldehyde.

19. The ketimine of Claim 16 in which the ketone is 2-butanone, 3,3-dimethyl-2-butanone, 2-pentanone, 3-pentanone, 2-methyl-3-pentanone, 2,4-dimethyl-3-pentanone or 5-methyl-2-hexanone.

20. The ketimine of Claim 18 in which the ketone is 4-methyl-2-pentanone.
